# EUROPEAN PATENT SPECIFICATION

(11) **EP 1 622 562 B1**
(45) Date of publication and mention of the grant of the patent: **15.09.2010**
(21) Application number: 04732243.3
(22) Date of filing: 11.05.2004
(51) Int. Cl.: A61H 31/00, A61N 1/38, A61N 1/39

(54) **SYSTEM FOR TREATING CARDIAC ARREST**
SYSTEME ZUR BEHANDLUNG VON HERZSTILLSTAND
SYSTEME POUR LE TRAITEMENT D'UN ARRET CARDIAQUE

(30) Priority: 12.05.2003 US 470229 P; 06.06.2003 US 456088
(43) Date of publication of application: 08.02.2006
(73) Proprietor: Jolife AB, 223 70 Lund (SE)
(72) Inventor: STEEN, Stig, S-226 50 Lund (SE)
(74) Representative: von dem Borne, Andreas
(86) International application number: PCT/SE2004/000712
(87) International publication number: WO 2004/112683

(56) References cited:
- US-A- 2 071 215
- US-A- 4 273 114
- US-A- 4 928 674
- US-A1- 2002 026 131
- US-B1- 6 213 960
- US-B1- 6 312 399
- DATABASE INSPEC [Online] TSUJI T. ET AL.: 'Development of a cardiopulmonary resuscitation vest equipped with defibrillator', XP002992867 Database accession no. 6314129 & ENGINEERING IN MEDICINE AND BIOLOGY SOCIETY, PROCEEDINGS OF THE 20TH ANNUAL INT CONF. OF THE IEEE vol. 1, 29 September 1998 - 01 November 1998, HONG KONG, CHINA, pages 426 - 427

## Description

### FIELD OF THE INVENTION

The present invention relates to an apparatus for treating sudden cardiac arrest. In particular, the present invention relates to the treatment of cardiac arrest triggered by ventricular fibrillation, asystolia, and PEA (Pulseless Electrical Activity).

### BACKGROUND OF THE INVENTION

Sudden cardiac arrest is commonly treated mechanically and/or by electrical defibrillation. Mechanical treatment comprises manual heart massage or, if available, mechanical chest compression followed by passive or active decompression, the length of a compression/decompression cycle typically being in the order of half a second to one second. A number of devices for mechanical stimulation of the heart under cardiac arrest are known in the art, such as the pneumatically driven LUCAS^{™} mechanical chest compression/ decompression system ("Lucas^{™} system"; an apparatus for compression and physiological decompression in Cardio-Pulmonary Resuscitation, CPR, manufactured by Jolife AB, Lund, Sweden). Specifically the Lucas^{™} system comprises a support structure for cardiopulmonary resuscitation of a patient and a compression/decompression unit. The support structure includes a back plate for positioning the patient's back posterior to the patient's heart, a front part for positioning around the patient's chest anterior to the heart, the front part having two legs, each leg having a first end pivotably connected to at least one hinge of the front part and a second end removably attachable to the back plate, the front part being devised to receive said compression/ decompression unit arranged to automatically compress or decompress the patient's chest when the front part is attached to the back plate. The compression/decompression unit comprises: a pneumatic unit arranged to run and control the compression and decompression; an adjustable suspension unit to which a compression/decompression pad is attached; and a handle by means of which the position of the pad can be controlled.

Defibrillation may be provided independently of and concomitantly with mechanical stimulation. An apparatus combining means for mechanical chest compression and defibrillation is disclosed in US 4,273,114 (Barkalow et al.).

US 6,312,399 B1 discloses a method for increasing cardiopulmonary circulation when performing cardiopulmonary resuscitation by repeated compression and decompression of the chest, comprising the electrostimulation of respiratory muscles to cause the patient to gasp to thereby cause an increase in the magnitude and duration of negative intrathoracic pressure during the decompression phase whereby the amount of venous blood flow into the heart and lungs is increased.

US 5,692,498 A discloses an endotracheal tube comprising a pressure-responsive valve in a first lumen of the tube which remains closed to prevent inflow of respiratory gas into the lungs until the pressure within the tube falls below a negative threshold intrathoracic pressure generated by chest compression and decompression on a non-breathing patient whereupon the valve opens to allow breathing gas under atmospheric pressure to flow to the lungs. Thereby the extent and duration of negative intrathoracic pressure during decompression is enhanced which is said to make peripheral venous blood flow into the heart and lungs when cardiopulmonary resuscitation is performed.

In cardiac arrest it is of utmost importance that adequate circulation be reestablished as soon as possible, that is within a few minutes from the onset of arrest. Any delay might lead to irreversible tissue damage. By "adequate circulation" is understood a circulation which is sufficient to protect vital organs and tissues from (further) damage, in particular by damage caused by insufficient oxygen supply.

### OBJECTS OF THE INVENTION

It is an object of the present invention to provide a means for carrying out the method.

The invention is as disclosed in the appended set of claims.

### SUMMARY OF THE INVENTION

It is disclosed a non-invasive method, which in not part of the invention, of re-establishing adequate circulation in a person under cardiac arrest. The method includes providing a breathing gas under positive pressure to the airways of the person and making the person inspire and expire against the positive pressure of the breathing gas for a period of time sufficient to establish a positive coronary perfusion pressure while mechanically stimulating the heart of the person by repeatedly compressing and decompressing the chest at a rate from 60 cycles per min to 200 cycles per min.

After applying the positive pressure of breathing gas and compression/decompression, electrostimulation may be used in form of pacing and/or defibrillation to the heart. Electrostimulation may be useful or provided only after the establishment of a positive coronary blood pressure even as mechanical stimulation and application of positive pressure gas is continued. The electrostimulation and mechanical stimulation may be applied in alternating periods or concurrently.

An important subsidiary feature is the provision of mechanical stimulation of the heart by a reciprocating pneumatic means, which is driven by the breathing gas used in the method. Particularly preferred is to use the breathing gas for inspiration by the patient after it has been used to drive the reciprocating pneumatic means, thus conducting the pressing gas after is has been vented from the reciprocating pneumatic means to the patient's airways. The breathing gas, preferably oxygen or a mixture of oxygen with inert gas of an oxygen content of preferably more than 50% by volume, is supplied in a compressed state from a gas tube or other pressurized gas source. The pneumatic means of the invention comprises a pad, disc, plate or similar which can be disposed so as to abut the breast of the patient.

It is preferred to provide the breathing gas by intubating the person or through a mask covering the mouth and/or nose of the patient. The proximal end of the intubation tube or the gas inlet of the mask is provided with the breathing gas from a gas container or gas line, preferably by a gas line putting the mask or intubation tube in communication with the gas exhaust of the aforementioned reciprocating pneumatic means.

A preferred compression depth is about 20% of the sternum depth, i.e., the distance of the sternum from a floor on which the patient rests with its back. The sternum depth varies in adult persons from about 175 mm to 260 mm; the preferred compression depth thus is from 35 mm to 52 mm according to the Guidelines by the European Resuscitation Council and the American Heart Association.

It is important that the compression and decompression be administered in a sudden manner, that is, the time from going from a fully compressed state to a fully decompressed state and vice-versa should be small compared to the length of a compression/decompression cycle, such as extending over a period corresponding to from about a sixth of a cycle to about a tenth of a cycle. In other words, the pressure curve in time/pressure diagram of the compression/decompression cycle should approach a trapezoidal wave curve. A compression/decompression cycle consists of a compression phase (CP) followed by a decompression phase (DCP). The compression phase (CP) consists of a dynamic portion (DNCP) during which the breast is being increasingly compressed until full compression is reached, and a static portion (STCP) in which the breast is kept in a state of full compression. Likewise the decompression phase (DP) consists of a dynamic portion (DNDCP) during which the breast is being increasingly decompressed until full decompression is reached, and a static portion (STDCP) in which the breast is kept in a state of full decompression such as in a physiologically decompressed state which the breast will naturally assume on decompression if allowed to do so.

It is preferred for the mean positive pressure of breathing gas in the lung to be from 1 mm Hg to 30 mm Hg during decompression, in particular from about 8 mm Hg to about 25 mm Hg, preferably about 20 mm Hg. Thus it is most important to maintain a positive pressure also during the decompression phase.

It is preferred to start the provision of electro-stimulation once a positive coronary perfusion pressure, in particular a positive coronary perfusion pressure of 10 mg Hg, more preferred of 15 mm Hg, even more preferred 25 mm Hg or more, has been obtained.

It is particularly preferred to provide defibrillating electro-stimulation (DF) during the last third of the static portion of the compression phase (STCP).

It is particularly preferred to provide pacing electro-stimulation (PC) within the time period from the start of the compression phase (CP) until the start of the decompression phase (DCP), more preferred during the second half of the dynamic portion of the compression phase (DNCP)and the first half of the static compression phase (STCP). Particularly preferred is to provide pacing electrostimulation within a time period of 0.10 to 0.15 seconds starting at the end of the dynamic compression phase (DNCP).

It is preferred for the rate of mechanical stimulation to be from 80 cycles/min to 120 cycles/min or 150 cycles/min, and even from 60 cycles/min to 200 cycles per min.

Preferably the period of time for exclusive mechanical stimulation is up to 180 seconds, preferably up to 30 seconds and more in the case of a preceding short period of asystolia, such as an asystolia lasting from 10 seconds to 50 seconds, and up to five minutes and more in the case of a preceding longer period of asystolia, such as an asystolia of two minutes and more. According to observations by the inventor the power required for compressing the breast to a given extent, such as from 30 mm to 45 mm or even up to 50 mm, will decrease by 25 per cent and more from that required at the start of compression within 20 to 60 seconds from the start of compression; the correct compression maintenance power required for continued compression can be estimated by measuring the compression resistance of the breast intermittently. It is preferred to accordingly control the driving power of the reciprocating pneumatic means by providing it with the correct amount of pressurized breathing gas. In this context "correct amount" of breathing gas refers to the amount of breathing gas required to maintain a desired compression/decompression profile, in particular essentially the same compression/ decompression profile over the entire treatment period. "Compression/decompression profile" designates the profile of the compression depth versus time curve. This sort of control will save compressed breathing gas.

Mechanical stimulation may be continued after the provision of electro-stimulation and re-establishment of circulation.

Electro-stimulation may be by defibrillation, such as in case of ventricular fibrillation.

Electro-stimulation may be provided in the form of pacing, such as in case of absence of ventricular fibrillation and presence of asystolia or bradycardia. Preferably pacing is provided at a constant basic rate, such as of about 80 beats per min; the pacing is made to follow the compression rate if the latter surpasses the basic rate.

Administration of electro-stimulation may be started once a minimum of 20 ml or more of blood has accumulated in the left ventricle in case of an adult person or a corresponding volume in a child.

Administration of electro-stimulation may be started once the time average-mean volume of blood in the right atrium at asystole or ventricular fibrillation has been reduced by 50% or more.

The breathing gas may be provided by intubation has a temperature below the ambient temperature, in particular a temperature of 5°C or even 10°C or more below the ambient temperature.

A cooling aqueous spray, such as cool saline may be provided to the patient by means of the breathing gas, which acts as a carrier.

A medicament alleviating the effects of cardiac arrest, such as the cardioplegic solution disclosed in WO 02/11741, and/or assisting the re-establishment of circulation, such as a vasodilator or adrenaline, may be administered to the patient by means of the breathing gas and/or an aqueous spray for which the breathing gas acts as a carrier.

It may be also provided an apparatus for resuscitating a person under cardiac arrest, the apparatus comprising a reciprocating means for mechanical stimulation of the heart; a means for the electric stimulation of the heart; a means for controlling the provision of mechanical and electric stimulation over time to allow electric stimulation to be provided once the time average-mean volume of blood in the right atrium at asystole or ventricular fibrillation has been reduced by 50% or more and/or 20 ml or more of blood has accumulated in the left ventricle in case of an adult person or a corresponding volume in a child and/or a positive coronary perfusion pressure, in particular a positive coronary perfusion pressure of 10 mg Hg, more preferred of 15 mm Hg, even more preferred 25 mm Hg or more, has been obtained; a tracheal tube or facial mask or an other means for adducing breathing gas under pressure to the airways of said person, and a source of compressed breathing gas for providing breathing gas to the person being resuscitated coupled with the tracheal tube or other breathing gas adducing means, and a means for controlling the pressure of the breathing gas provided to the person.

The source of compressed breathing gas may be designed to provide gas for driving the reciprocating means.

It is preferred for the reciprocating means to be capable of providing mechanical stimulation at a rate of from 60 cycles per min to 200 cycles per min., in particular from about 80 to about 120 cycles per min or more.

The means for controlling the provision of mechanical and electric stimulation may comprise the means for controlling the pressure of the breathing gas provided to the patient.

An administration means may be provided for administering to the person under resuscitation a medicament for alleviating the effects of cardiac arrest, such as the cardioplegic solution disclosed in WO 02/11741, and/or assisting the re-establishment of circulation, such as a adrenaline or noradrenaline, vasopressin, cortisone, insulin, cyclosporin A, the administration means being coupled to or integrated with the tracheal tube or other breathing gas adducing means and being capable of producing an aqueous mist or spray containing the medicament.

An apparatus for re-establishing circulation in a person under cardiac arrest is disclosed comprising a source of compressed breathing gas; means for providing said breathing gas under positive pressure to the airways of said person; pneumatic reciprocating means for mechanically stimulating the heart of said person by repeatedly compressing and decompressing the chest at a rate from 60 cycles per min to 200 cycles per min; electrical means for providing electrostimulation in form of pacing and/or defibrillation to the heart; wherein the compressing breathing gas is used for driving said reciprocating pneumatic means and wherein the breathing gas vented from the reciprocating pneumatic means is used for providing breathing gas under positive pressure to the airways of the person.

The means for providing breathing gas to the patient preferably comprises a facial breathing mask or a device for intubation of the persons's airways. Breathing gas may however also be provided to the patient by transtracheal intubation.

The electrical means for providing electrostimulation comprises two electrodes, a front electrode to be applied on the breast of the patient adjacent to the pad, plate or disc, etc. of the reciprocating means or integrated in the pad, plate or disc, etc., and a rear electrode to be applied to the back of the patient. The breast electrode is preferably disposable. The rear electrode is preferably integrated into a back plate on which the patient may rest; it is integrated in such manner that it will be disposed about perpendicularly under the sternum if the patient is resting in a correct position on the back plate. Advantageously the electrical means for providing electrostimulation includes a means for analyzing ECG signals, which are picked up by the electrodes and conducted to the ECG analyzing means.

In the first place, the pacing frequency is adapted to follow the frequency of mechanical compressions/ decompressions, and is controlled accordingly. This is particularly important as long as the (unpaced) cardiac rate of the patient is slower than the frequency of mechanical compressions/decompressions. However, once the cardiac rate of the patient exceeds the set frequency of mechanical compression/decompression, it may be used to control that frequency. Providing mechanical compressions at the pulse rate will make the contraction of the heart more vigorous. In case of Pulseless Electrical Activity (PEA) the rate of mechanical compression/ decompression can be controlled by, for instance, pulse oxymetry so as to provide for optimal oxygen saturation of the patient's blood. It is furthermore within the scope of the invention to determine the optimal point in time for an electrical stimulus to the heart within a cardiac cycle by empirically scanning the effect of such provision at selected points in time over several cycles. In the case of PEA there is, however, no reason for stimulating the heart in the QT-phase of the ECG. It is also within the scope of the invention to provide the apparatus of the invention with an ultrasound imaging device for visualizing the contractions of the heart; alternatively this information can also be used for controlling the administration of electrical defibrillating or pacing signals to the heart.

According to the present invention is also disclosed a facial mask provided comprising a disposable flexible plastic tube which is directly or indirectly connectable to the downstream side of a valve manifold pertaining to a reciprocating compression/ decompression device for resuscitation driven by compressed breathing gas.

Furthermore, according to the present invention is disclosed a disposable endotracheal tube provided, at its proximal end, with a flexible plastic tube, which is directly or indirectly connectable to the downstream side of a valve manifold pertaining to a compression/decompression device for resuscitation.

Additionally is disclosed a reciprocating compression/decompression device for resuscitation of a person driven by compressed breathing gas, which comprises a detachable means for administering a portion of the breathing gas consumed for driving the device to the airways of said person at a positive pressure over a time period of 1 min or more, in particular of 5 min or more. Preferably the breathing gas comprises at least 50% by volume of oxygen. The detachable means is selected from facial mask, endotracheal tube or tube for exotracheal intubation.

The invention will now be explained in more detail by reference to a preferred embodiment illustrated by a drawing.

### DESCRIPTION OF THE FIGURES

In the drawing,

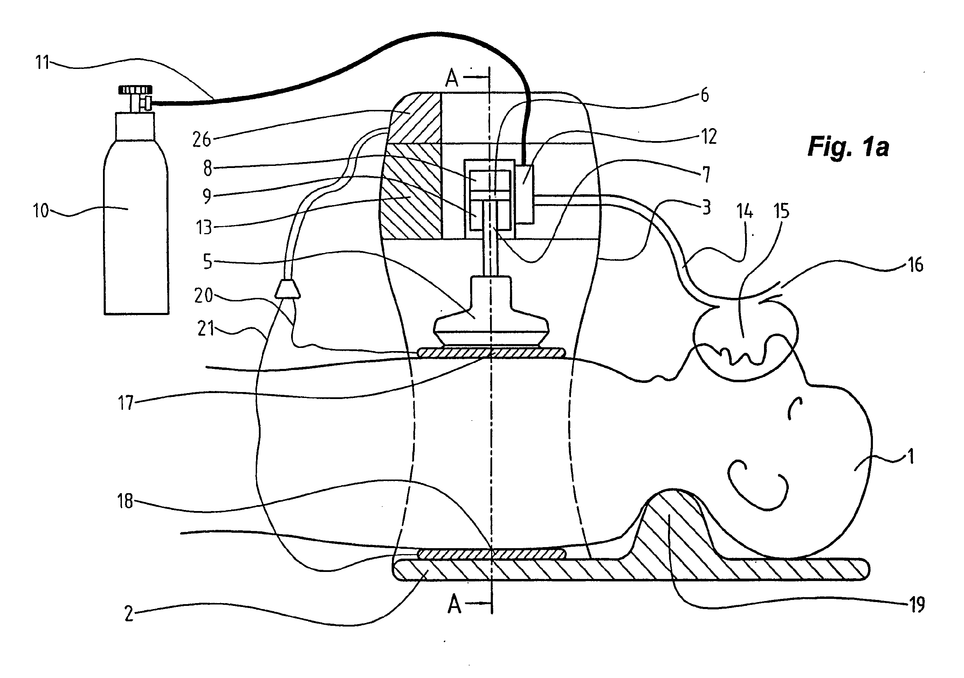

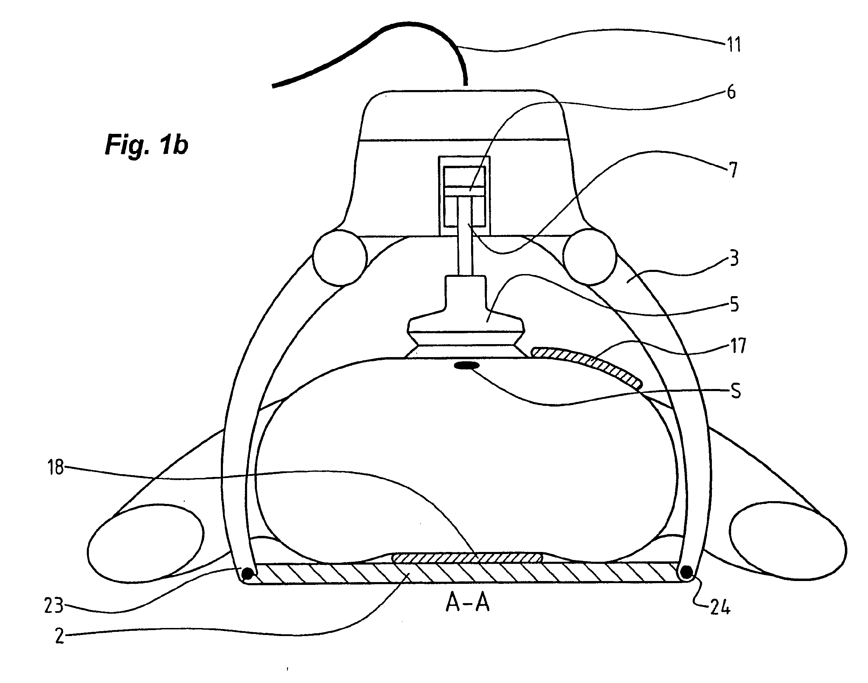

- Fig. 1 a: is a schematic side view of the apparatus of the invention applied to a person in cardiac arrest;
- Fig. 1 b: is a schematic section A-A of the apparatus of Fig. 1;
- Fig. 2: is a flow sheet illustrating the treatment of cardiac arrest;
- Fig. 3: is a flow sheet of an ECG diagnosis comprised by a method, and of available therapeutic choices in consideration of the diagnostic result;
- Fig. 4: is a schematic representation of the driving and control of the apparatus of the invention, in form of a block diagram;
- Fig. 5: is a schematic representation of the driving of the pneumatic reciprocating means and the pressure control of the breathing gas vented from the reciprocating means to be provided to the patient's airways at a positive pressure, in form of a block diagram;
- Fig. 6: is a diagram illustrating the provision of mechanical compression over time to an adult person and the concomitant provision of pacing or defibrillation.

### DESCRIPTION OF PREFERRED EMBODIMENTS

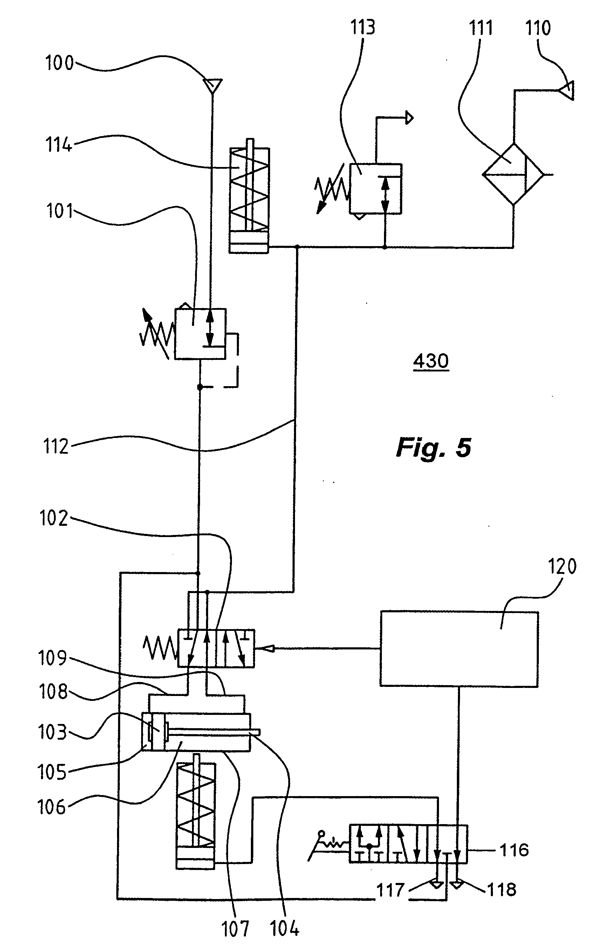

**EXAMPLE 1. Apparatus for re-establishing circulation in a person under cardiac arrest.** The apparatus for re-establishing circulation in a person under cardiac arrest according to the invention is illustrated schematically in Figs. 1 a and 1 b. It is based on and comprises a Lucas^{™} transportable compression/decompression system (TCDC system; Jolife AB, Lund Sweden). The TCDC system comprises a back plate 2 and a yoke or front part 3. A patient 1 in cardiac arrest is placed in a supine position on the back plate 2 such that the center of the back plate 2 is disposed under the sternum S. The back plate 2 extends laterally on both sides of the patient. One of the extensions is provided with a snap lock 23 which allows the free end of the yoke 3, which is hingedly mounted at the other extension (at 24), to be quickly fixed at the back plate 2. Thereby the breast of the patient becomes enclosed by the TCDC unit which, in a transverse section (Fig. 1 b), is similar to a stirrup. The central section of the yoke 3 is disposed at a distance from the sternum S. The central section of the yoke 3 includes a pneumatic compression/ decompression unit which comprises a compression/decompression pad 5 connected to a piston 6 in a twin chamber 8, 9 of a pneumatic cylinder by a rod 7. Compressed air or oxygen or other breathing gas from a gas cylinder 10 or a line for such gas is alternatingly fed to the chambers 8, 9 of the pneumatic cylinder via a flexible reinforced plastic tube 11 and the inlet valves of a valve manifold 12. The inlet and corresponding outlet of the manifold 12 are controlled by a main control unit 13 comprising a rechargeable battery and microprocessor. The main control unit controls, i.a., the reciprocating rate of the piston 6 and the compression/decompression pad 5. They are disposed to reciprocate in a dorsal/ventral direction at a desired rate which can be varied from 60 strokes per min to 150 strokes per min. At the circumference of its face abutting the breast of the patient the pad 5 is provided with a adhesive material to make (not shown) it adhere to the skin. This causes the breast to be physiologically decompressed during the ventral direction phase of the reciprocating movement. The pressure of the oxygen gas leaving the chambers 8, 9 of the pneumatic cylinder is controlled, that is, kept about constant at a desired positive pressure by means of the breathing gas pressure control circuitry illustrated in Fig. 5 which is described in more detail below. From the outlet valves at the manifold 12 the breathing gas is conducted through a flexible tube 14 to a facial mask 15 disposed over a central portion of the patient's face comprising the nose and mouth, and having an outlet 16. Thereby the patient is continuously provided with fresh breathing gas, if so desired under a slight positive pressure. The provision of breathing gas at a fully controlled positive pressure by the arrangement of Fig. 5, is particularly important if the breathing gas is administered via intubation; when administered by means of a breathing mask the control circuitry in Fig. 5 can be partially or fully dispensed with if the pressure in the mask is controlled by the width of the outlet of the mask or in a similar manner.

Adjacent to the pad 5 is disposed a front (breast) electrode 17 which abuts the breast of the patient and is secured by an adhesive. It may be also or additionally held against the breast by a spring or similar elastic element attached to the yoke 3. It is also conceivable to integrate the front electrode 17 into the face of the pad 5 facing the breast of the patient. A second, rear (back) electrode 18 is mounted on the upper (inner) face of the back plate 2. The electrodes 17, 18 serve as ECG electrodes and as pacing and defibrillating electrodes. They are connected to an electrode control unit 26 by means of electric conductors 20, 21. The electrode control unit 26 is capable of carrying out simple ECG measurements to discern between asystole and ventricular fibrillation, of dispensing defibrillation bursts of electric energy, and of pacing the heart at a desired rate. The electrodes 17, 18 serve as electrodes for ECG measurements, defibrillation, and pacing. The electrode control unit 26 also comprises a source of electric energy in form of a rechargeable battery which it may share with the main control unit 13. As a safeguard measure the control unit may be supplied with electricity from the mains by means of an integrated transformer/rectifier unit.

The back plate 2 which is provided with a neck support 19 is disposed under the patient's neck. The height of the neck support 19 is sufficient to make the patient's head fall back until it comes to rest against a superior end portion of the back plate 2. As will be explained below the positioning of the patient's head in this manner facilitates safe intubation.

**EXAMPLE 2. Endotracheal tube.** An endotracheal tube suitable for use in the invention is disclosed in WO 94/00174. It comprises a flexible plastic tube, a flexible tubular tip made of molded material axially joined to the flexible tube, and an inflatable balloon consisting of a tubular sleeve positioned on the endotracheal tube and the tubular tip and communicating with a balloon inflating/deflating system near the proximal end of the endotracheal tube, the inflatable balloon and the balloon inflating/deflating system communicating via a channel extending longitudinally through the wall of the flexible tube. An endotracheal tube of this design is marketed by Laboratories Pharmaceutiques VYGON, Ecouen, France under the trade name "Boussignac adult entdotracheal tube". It is important to provide a tracheal tube having a breathing gas channel lumen which is as wide as possible in relation to the outer diameter of the tube, such as comprising 90 per cent or more of the tube area in a transverse section.

In case of active decompression to a physiological decompressed position (that is, by avoiding over-decompression) a vigorous flow of breathing gas trough the tracheal tube into the lungs is observed. This gives rise to a positive pressure of about 8 mm Hg (at a breathing gas flow of about 15 L/min) in the heart when decompressing with the Lucas® device compared with a negative pressure from zero to about -5 mm Hg in the case of passive (manual or other) decompression. If the flow through the tracheal tube is increased to about 25 L/min the positive pressure at decompression rises to about 15 mm Hg. During compression the Lucas® device itself creates a positive pressure of about 15 to 20 mm Hg. Since the lungs can be damaged by breathing gas pressures of 40 mm Hg and above the flow of breathing gas through a tracheal tube in active decompression with the Lucas® device should be limited to 20 L/min.

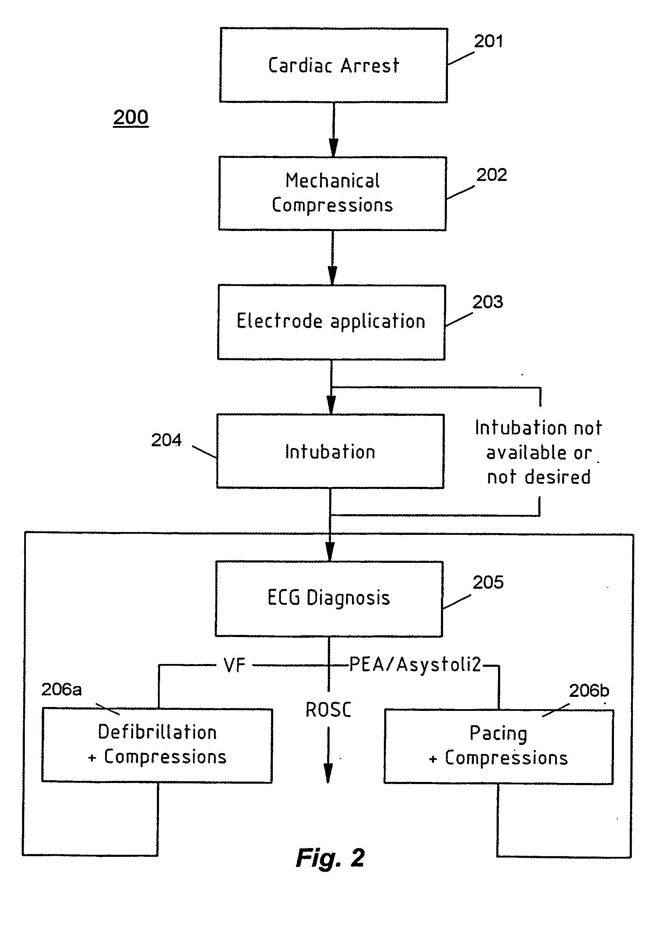

**EXAMPLE 3. Resuscitation.** Now reference is made to the flow sheet 200 in Fig. 2. A patient in cardiac arrest state 201 is administered mechanical breast compressions 202 as soon as possible after the onset of arrest. Initially the compressions 202 may have to be given by hand. When the apparatus of the invention has been mounted on the patient mechanical compression is immediately started. At the same time or shortly thereafter the measurement of the electric activity of the heart is started by means of application 203 of electrodes 17, 18. The electric signals received by the electrodes 17, 18 are processed in the CPU to detect by ECG 203 whether the patient's heart is in an asystolic (EMD, electro mechanical dissociation) or ventricular fibrillation (VF) state or pulseless electric activity state or a normal state (in this context a "normal" state designates a state which does not require defibrillation or pacing but which is not necessarily a healthy state). Thereafter the patient may be intubated 204, if desired. ECG diagnosis reveals whether the patient has ventricular fibrillation (VF) or PEA (Pulseless Electrical Activity) or asystolia. In the first case 206a electrical defibrillation pulses are administered through electrodes 17, 18 while continuing with mechanical compression/ decompression. In the second case 206b electrical pacing pulses are administered through electrodes 17, 18 while continuing with mechanical compression/ decompression. Intermittently ECG diagnosis 205 is repeated to monitor the effect of the treatment and to form a basis for further therapeutic decisions.

In case of ventricular fibrillation compressions with or without physiological decompression and defibrillation 206a will be administered until the return of spontaneous circulation (ROSC). Thereafter compressions 202 will be continued to be administered, optionally accompanied by pacing 206b. A suitable defibrillator which can be incorporated into the apparatus of the invention is the Heartstart FR2 AED (Leardal Medical A/S, Stavanger, Norway).

In case of asystolia/PEA compressions 202 with or without physiological decompression will be administered in combination with pacing 206b at least until ROSC is observed but preferably for a substantially longer time such as, for instance, until the patient is under hospital care.

Depending on the circumstances, such as the presence or absence of persons capable of carrying out intubation 204, the patient will be intubated or not. Intubation 204 may be used to administer pharmaceutical agents to the patient, such as an aqueous solution of potassium chloride. It is also feasible to provide this or other aqueous solution of pharmacologically active agents in a cold state, that is substantially below room temperature, such as at 10° C or lower.

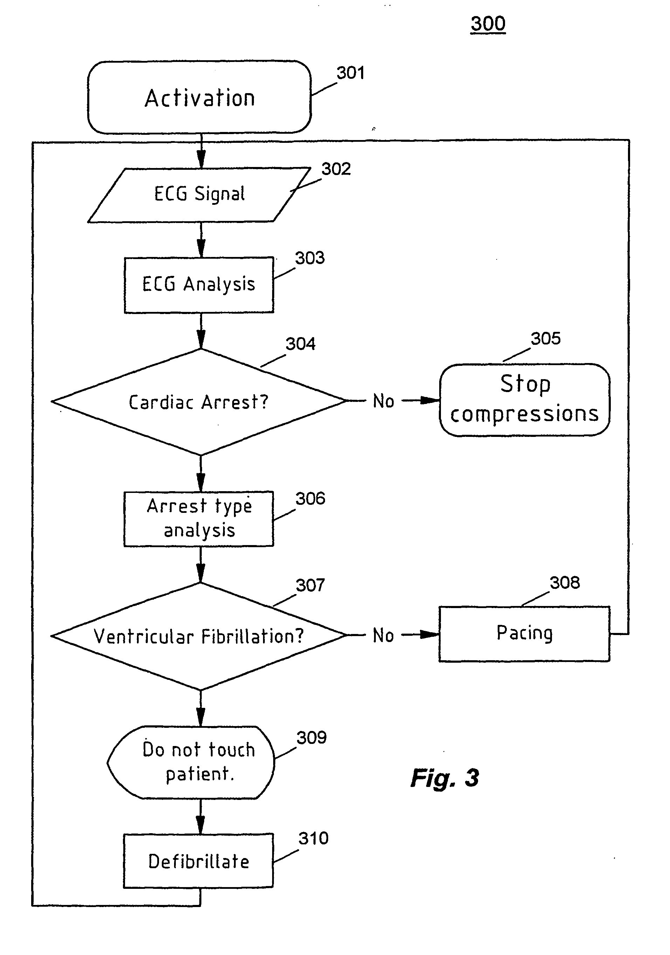

**EXAMPLE 4. Therapeutic decisions.** For the sequence of therapeutic decisions pertaining to the method reference is made to the flow sheet 300 in Fig. 3. After activation 301 of the apparatus of the invention, that is, during the administration of compressions/ decompressions 202, the ECG signal 302 of the patient is measured and analyzed 303. The first therapeutic decision 304 is based on whether the patient is under cardiac arrest or not, since some patients may seem to be under cardiac arrest while actually being in a state in which the heart is only beating faintly, such as in a state in which the body temperature of the patient is several degrees below the normal body temperature.

Specifically, as illustrated in Fig. 3, the ECG signal is analyzed for cardiac arrest. If a "normal" though slow pulse is detected there is not cardiac arrest, and compression/decompression consequently is immediately stopped 305. If there is cardiac arrest, the type of arrest is determined in the next step in which the ECG signal is analyzed 306 for ventricular fibrillation. If an ECG signal pattern characteristic of ventricular fibrillation 307 is detected the patient is defibrillated 310. If no such signal pattern is detected electric pacing pulses 308 are administered to the patient.

The foregoing therapeutic decisions can be taken automatically by the apparatus if so required. This increases its usefulness in the hands of persons, which are lacking adequate medical training. On the other hand, the apparatus provides for display of the diagnostic result of each step to permit to the user to initiate the most adequate treatment in a specific situation. During defibrillation 310 the medical personnel involved must not touch 310 the patient.

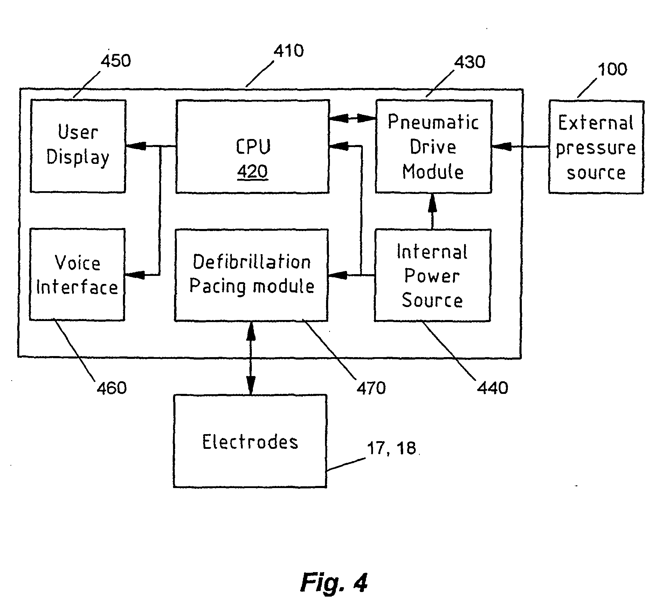

**EXAMPLE 5. Apparatus control and driving.** The principles of apparatus control and driving are illustrated in Fig. 4. The apparatus is controlled by a microprocessor (CPU) 420 comprising a data storage means in which appropriate software is stored, such as software for digitalizing electrode signals, software for comparing the digitalized signals with signals representative of various types of heart activity, and software for controlling the pneumatic and electric means of the apparatus. The control unit 410 is powered by an internal power source 440, such as a rechargeable battery. The control unit 410 comprises a user display 450 for reading electrode measurement and other data. It may also comprise a voice interface 460 by which standard instructions to the personnel may be given by the CPU 420 such as, for instance, instructions regarding the need to replace the breathing gas source, etc.

The control and driving 410 unit further comprises a defibrillation and pacing module 470 including a transformer. An ECG analysis function is integrated in the defibrillation and pacing module. Suitably a commercial defibrillator such as the Cardio-Aid Model 200 defibrillator (Artema Medical AB, Stockholm, Sweden; company acquired in 2001 by Cardiac Science, Inc., U.S.A.) provided with a pacer unit may be incorporated in the apparatus of the invention.

Additionally the control and driving unit includes a pneumatic drive module 430 the functional design of which is shown in Fig. 5. It is fed with pressurized oxygen gas from a breathing gas grade oxygen flask 100. By a pressure reduction and control valve unit 101 the pressure of the breathing gas is reduced and kept about constant. From there the pressurized oxygen gas is conducted to a valve manifold 102 the valves of which are controlled by the apparatus control unit 120 (e.g., CPU 420). Alternatingly the pressurized oxygen gas is fed via lines 108, 109 to either side (105, 106) of a piston 103 in a pneumatic cylinder 107 to cause the piston 103 to move in a reciprocating manner. By a rod 104 the movement of the piston 103 is transferred to a pad so as to alternatingly compress and decompress the breast of a patient (not shown). The substantially but not fully decompressed gas leaves the chambers of 105, 106 of the pneumatic cylinder 107 by exhaust valves of the manifold 102 and is conducted from there to the proximal end (at 110) of one of the channels of an intubation tube by a gas line 112 comprising an attenuator 114, a safety valve 113 and a reservoir 111. The pressure of the oxygen gas fed to the manifold 110 may be further controlled via the control unit 120 by an attenuation circuit 116, 117, 118, which is not further explained.

**EXAMPLE 6. Endotracheal tube.** An endotracheal tube suitable for use in the invention is disclosed in WO 94/00174. It comprises a flexible plastic tube, a flexible tubular tip made of molded material axially joined to the flexible tube, and an inflatable balloon consisting of a tubular sleeve positioned on the endotracheal tube and the tubular tip and communicating with a balloon inflating/deflating system near the proximal end of the endotracheal tube, the inflatable balloon and the balloon inflating/deflating system communicating via a channel extending longitudinally through the wall of the flexible tube. An endotracheal tube of this design is marketed by Laboratories Pharmaceutiques VYGON, Ecouen, France under the trade name "Boussignac adult entdotracheal tube".

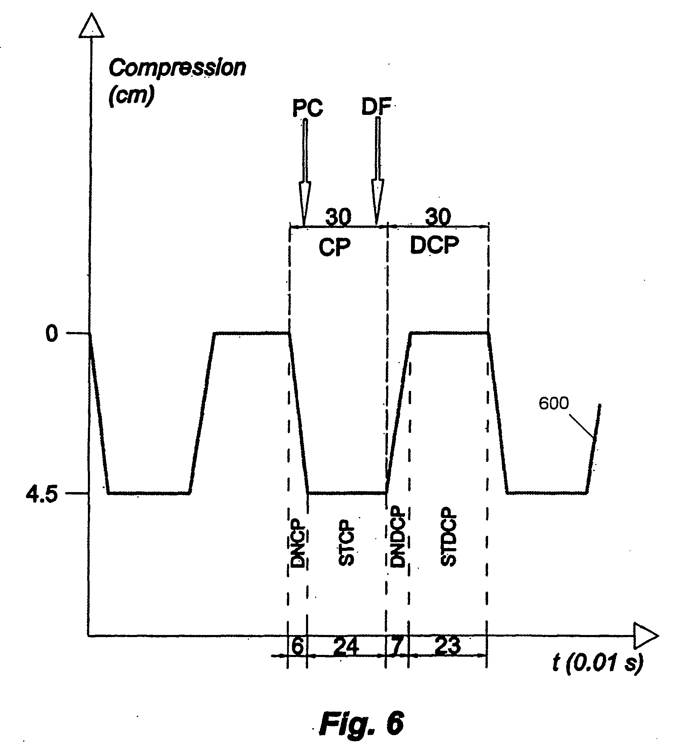

**EXAMPLE 7.** The diagram in Fig. 6 illustrates the provision of mechanical compression over time to an adult person and the concomitant provision of pacing or defibrillation (PC, pacing electro-stimulation; DF, defibrillating electro-stimulation; CP, compression phase; DCP, decompression phase; DNCP, dynamic portion of compression phase; STCP, static

portion of compression phase; DNDCP, dynamic portion of decompression phase; STDCP, static portion of decompression phase) The compression curve 600 has a trapezoidal wave shape.

The following three case reports are given to further illustrate various elements of the invention.

**Case report no. 1.** Woman, 73 yrs. Had a coronary bypass surgery 5 years ago and a myocardial infarction 3 years ago. Collapsed during a concert (in the following, indications of time relate to the time of collapse). A physician who happened to be present found no palpable pulse. He started CPR at once (15 chest compressions and 2 mouth-to-mouth inblowings per min). An ambulance with a cardiopulmonary resuscitation team arrived at 6 min. They placed the patient in a LUCAS device and started chest compression. At 7.5 min the ECG indicated ventricular fibrillation. The patient was then intubated via the mouth with a tracheal tube (Boussignac tube), the oxygen channel in the tube being connected to the oxygen supply tube of the LUCAS^{™} device. 15 L of gas per min were given distally of the Boussignac tube as continuous insufflation of oxygen (CIO). At 8.5 min a pulse oxymeter was connected to one fingertip. Pulses with oxygen saturation of 100% were observed, the patient's skin had reddish appearance indicating good skin circulation. Good pulses could be palpated in the carotid artery and even in the groin. On the ECG display ventricular fibrillation was seen to become increasingly coarse. At 9 min of LUCAS-CPR one defibrillation shock (360 J) was given during on-going compression. The patient turned asystolic, but after about 10 sec, a sinus rhythm with a frequency of 60 per min was seen. Mechanical compression was continued until the frequency had risen to 90 per min at 12 min. The device was then turned off. Within 1 min the pulse weakened and the oxymetric value decreased from 100 to 70. Compressions were again administered, the patient moved to a stretcher and carried to the ambulance while continuing with LUCAS-CPR and CIO treatment. At arrival at the hospital's emergency unit (at about 25 min) the pulse oxymetric saturation had increased to 100%. An arterial needle was put into the radial artery and a central venous catheter was put in place by using Seldinger's technique via the external jugular vein. Blood gases showed that arterial oxygenation was better than normal (PaO₂ = 49), pH 7.45, PaCO₂ 3.5. Base excess -1. The device was now turned off. The patient has sinus rhythm with a frequency of 90, and she keeps a blood pressure of 110/70, mean 90. The patient was taken to the intensive care unit and put under close observation for 24 h. No signs of myocardial infarction were observed. The patient was stable and fully conscious. Next day she was taken off emergency monitoring; after one week in normal care she was sent home. On a control 1 month later the patient was doing fine.

**Case report no. 2:** Man, 36 yrs. Was found without signs of life in a park early in the morning at an ambient temperature of-1°C. An ambulance arrived at about 5 min. The patient was without pulse. No respiration was seen. The ECG showed extreme bradycardia at 2-3 beats per min. The temperature of the patient was 22°C. The chief ambulance physician was phoned in his home. He rushed to the site with a LUCAS^{™} device. When seeing the patient's small pupils he decided to start resuscitation by placing the patient in the device and starting compressions. The patient then was intubated with a Boussignac tube and CIO at the distal end of the tube established by connecting the oxygen channel of the tube to the oxygen exhaust line of the LUCAS^{™} device. The low voltage ECG of the device indicated that the patient had a sinus rhythm with a frequency of 3 to 5 per min. Good pulses now could also be palpated in the carotid artery. The patient was transported in the working device to the hospital where he was taken directly to the operating theatre and connected to a heart-lung machine via the femoral vessels. He was slowly warmed up to 32°C. At this stage his heart was beating vigorously at 70 per min, producing a pressure of 110/60. After weaning from the heart-lung machine he was taken to the intensive care unit and allowed to warm up spontaneously to normothermic temperature over the following 6 hours. After 1 week he could leave the hospital fully intact.

**Case report no. 3:** Man, 48 yrs. Collapsed in his office at 10 o'clock in the morning. The heart ambulance was called immediately, and arrived at 4 min. Up to then no resuscitation had been started. The ambulance personnel consisted of 2 paramedics and a driver. The chief paramedic-immediately checked for a pulse in the carotid artery with a negative result. Compression of the skin produced a pale spot indicating circulatory arrest. The patient was immediately placed in the LUCAS^{™} device and mechanical chest compression was started within 30 sec. The ECG showed the patient to be asystolic. One paramedic lifted the cheeks of the patient to keep the airway free, and started ventilation with a Rubens self-expanding bag connected to the oxygen exhaust channel of the Lucas^{™} device, insufflating the Rubens bag with about 15 L per min of oxygen. The Rubens bag has a pressure safety valve controlling the pressure in the bag not to exceed 30 cm of water column. In this manner the patient was ventilated with 100% oxygen at a compression/decompression frequency of 100 per min. A pulse oxymeter placed on the fingertip showed good pulses with 98% oxygen saturation. Ventilation was then given more aggressively which made the saturation rise to 100%. The ECG indicated the patient to be still asystolic. Now the pacemaker function of the LUCAS^{™} device was activated, and the heart paced during each compression. Pulses in the carotid artery soon could be easily felt. At the start of resuscitation the patient's pupils were dilated but now became constrict. The compressions were stopped, and only the pacemaker function was retained. Since the pulse started to weaken and the saturation to fall the device was started again which made oxygen saturation immediately return to 100%. An ambulance with an anaesthetic team (physician and nurse) arrived about 7 min later to see a well-oxygenated and well-circulated patient with the device working at 100 compressions/ decompressions per min and a pacemaker ECG with a frequency of 100. Upon stopping the device a fall in oxygen saturation was immediately seen. The device was started again, mask ventilation was stopped, and the patient was intubated with a Boussignac tube the oxygen channel of which was connected to the oxygen exhaust tube from the LUCAS^{™} device. An oxygen flow of 25 liter per min was provided at distally of the Boussignac tube to the tracheal tube. The frequency of compression was increased to 120 per min while continuing pacing at 100 per min. After 5 min pacing was stopped, and the compression frequency reduced to 80 per min. Now a sinus rhythm with a frequency of about 65 per min could be seen. An arterial needle was placed in the radial artery, the compressions were discontinued, and an arterial pressure of 115/80 measured. The patient was put on a stretcher while keeping the device mounted a decompressed state continuing ECG monitoring. During the transport to the hospital an extreme bradycardia was observed. Systolic blood pressure fell to less than 60. Compressions (100 per min) were re-started a frequency of 100, and the pacemaker function activated. When arriving at the hospital the compressions as well as pacing were stopped. The ECG now showed a heart block grade III and a pulse frequency of about 35. Compression and pacing (at 100 per min) was immediately re-started. The arterial pressure quickly rose to 120/80 while full oxygen saturation was obtained. The patient was taken to the operating theatre and provided with a transvenous pacemaker in the right ventricle and one electrode in the right atrium, upon which internal pacing was started. During the insertion of the pacemaker electrode the device was working at 100 compressions and 100 beats per min. After the permanent pacemaker had been implanted as indicated above, the compressions and pacing by the Lucas device were stopped. The patient was now in a stable condition with a pacemaker rhythm of 100 per min. He was taken to the intensive care unit and put in a ventilator until next day. After extubation he was fully awake but totally amnesic in regard of the incident. After one week he left the hospital fully intact.

## Claims

1. An apparatus for re-establishing circulation in a person under cardiac arrest comprising a source (10) of compressed breathing gas; means (12, 14, 15) for providing said breathing gas under positive pressure to the airways of said person; pneumatic reciprocating means (6) for mechanically stimulating the heart of said person by repeatedly compressing and decompressing the chest at a rate from 60 cycles per min to 200 cycles per min; electrical means (17, 18, 26) for providing electrostimulation in form of pacing and/or defibrillation to the heart; wherein the pneumatic reciprocating means (6) is driven by said compressed breathing gas; **characterized in that**
the apparatus comprises means (12, 14, 15) for adducting breathing gas vented from the reciprocating means (6) under positive pressure to the airways of said person, which means (12, 14, 15) comprise a valve manifold (12, 102) the valves of which are controlled by a control unit (120), wherein the control unit (120) controls the pressure of the breathing gas vented to the airways of the person in a manner that the pressure of the breathing gas vented to the
airways of the person is kept about constant at the desired positive pressure.

2. The apparatus of claim 1, wherein the means (12, 14, 15) for providing breathing gas to the patient comprises a facial breathing mask (15) or a device for intubation of the person's airways.

3. The apparatus of claim 1 or 2, wherein the pneumatic reciprocating means comprises a pad (5), plate, disc or similar element disposable so as to abut the breast of the patient, and wherein the electrical means (17, 18, 26) for providing electrostimulation comprises two electrodes (17, 18), a front electrode (17) to be applied on the breast of the patient, the front electrode (17) being positionable adjacent to the pad (5), plate, disc or similar element of the reciprocating means or being integrated therein in a manner making it face the breast and being abutable thereto in combination with the pad (5), plate, disc or similar element, and a rear electrode (18) disposed so as to be abutable to the back of the patient.

4. The apparatus of claim 3, wherein the electrical means (17, 18, 26) for providing electrostimulation includes a means (26) for analyzing ECG signals picked up by said electrodes (17, 18) and conducted to the ECG analyzing means.

5. The apparatus of claim 1 to 4, wherein the pacing frequency is controlled to follow the frequency of mechanical compression/decompression.

## Patentansprüche

1. Vorrichtung zum Wiederherstellen des Kreislaufs einer Person bei Herzstillstand, umfassend eine Quelle (10) von komprimiertem Atmungsgas; Mittel (12, 14, 15) zum Bereitstellen des Atmungsgases unter Überdruck für die Atemwege der Person; pneumatisches hin- und hergehendes Mittel (6) zum mechanischen Stimulieren des Herzens der Person durch wiederholtes Komprimieren und Dekomprimieren des Brustkorbs bei einer Rate von 60 Zyklen pro Minute bis 200 Zyklen pro Minute; elektrisches Mittel (17, 18, 26) zum Erzeugen einer Elektrostimulation in Form einer Schrittsteuerung und/oder Defibrillierung am Herzen; wobei das pneumatische hin- und hergehende Mittel (6) durch das komprimierte Atmungsgas angetrieben wird; **dadurch gekennzeichnet, dass**
die Vorrichtung Mittel (12, 14, 15) zum Zuführen von Atmuzlgsgas, das von dem hin- und hergehenden Mittel (6) abgezogen wird, unter Überdruck zu den Atemwegen der Person umfasst, wobei das Mittel (12, 14, 15) einen Ventilverteiler (12, 102) umfasst, dessen Ventile von einer Steuereinheit (120) gesteuert werden, wobei die Steuereinheit (120) den Druck des Atmungsgases, das den Atemwegen der Person zugeführt wird, derart steuert, dass der Druck des Atmungsgases, das den Atemwegen der Person zugeführt wird, annähernd konstant bei dem gewünschten Überdruck gehalten wird.

2. Vorrichtung nach Anspruch 1, wobei das Mittel (12, 14, 15) zum Bereitstellen von Atmungsgas für den Patienten eine Gesichtsatmungsmaske (15) oder eine Vorrichtung zur Intubation der Luftwege der Person umfasst.

3. Vorrichtung nach Anspruch 1 oder 2, wobei das pneumatische hin- und hergehende Mittel ein Polster (5), eine Platte, eine Scheibe oder ein ähnliches Element umfasst, das so angeordnet werden kann, dass es auf dem Brustkorb des Patienten liegt, und wobei das elektrische Mittel (17, 18, 26) zum Bereitstellen einer Elektrostimulation zwei Elektroden (17, 18) umfasst, eine vordere Elektrode (17), die an dem Brustkorb des Patienten angelegt wird, wobei die vordere Elektrode (17) neben dem Polster (5), der Platte, der Scheibe oder dem ähnlichen Element des hin- und hergehenden Mittels positionierbar ist oder darin in einer weise integriert ist, dass sie dem Brustkorb zugewandt ist und auf diesem in Kombination mit der Platte, der Scheibe oder dem ähnlichen Element aufgelegt werden kann, und eine hintere Elektrode (18), die so angeordnet ist, dass sie am Rücken des Patienten angelegt werden kann.

4. Vorrichtung nach Anspruch 4, wobei das elektrische Mittel (17, 18, 26) zum Bereitstellen einer Elektrostimulation Mittel (16) zum Analysieren von ECG-Signalen enthält, die von den Elektroden (17, 18) aufgekommen und zu dem ECG-Analysierungsmittel geleitet werden.

5. Vorrichtung nach Anspruch 1 bis 4, wobei die Schrittsteuerungsfrequenz so gesteuert ist, dass sie der Frequenz einer mechanischen Komprimierung/Dekomprimierung folgt.

## Revendications

1. Appareil destiné à rétablir la circulation chez un patient en arrêt cardiaque, qui comprend une source (10) de gaz respiratoire comprimé ; un moyen (12, 14, 15) pour alimenter les voies respiratoires dudit patient en ledit gaz respiratoire sous pression positive ; un moyen pneumatique animé d'un mouvement de va-et-vient (6) destiné à mécaniquement stimuler le coeur dudit patient par compression et décompression répétées du thorax à un taux de 60 cycles par minutes à 200 cycles par minute ; un moyen électrique (17, 18, 26) destiné à fournir une électrostimulation sous la forme d'une stimulation et/ou d'une défibrillation au coeur ; le moyen pneumatique animé d'un mouvement de va-et-vient (6) étant entrainé par ledit gaz respiratoire comprimé ; **caractérisé en ce que** l'appareil comprend un moyen (12, 14, 15) d'adduction de gaz respiratoire ventilé par le moyen animé d'un mouvement de va-et-vient (6) sous pression positive aux voies respiratoires dudit patient, lequel moyen (12, 14, 15) comprend un collecteur à vannes (12, 102) dont les vannes sont commandées par une unité de commande (120), l'unité de commande (120) commandant la pression du gaz respiratoire ventilé dans les voies respiratoires du patient de manière à ce que la pression du gaz respiratoire ventilé aux voies respiratoires du patient soit maintenue approximativement constante à la pression positive souhaitée.

2. Appareil selon la revendication 1, le moyen (12, 14, 15) de fourniture de gaz respiratoire au patient comprenant un masque respiratoire facial (15) ou un dispositif d'intubation des voies respiratoires du patient.

3. Appareil selon la revendication 1 ou 2, le moyen pneumatique animé d'un mouvement de va-et-vient comprenant un coussinet (5), une plaque, un disque ou élément similaire jetable destiné à venir reposer contre la poitrine du patient, et ledit moyen électrique (17, 18, 26) destiné à fournir une électrostimulation comprenant deux électrodes (17, 18), une électrode avant (17) destinée à être appliquée sur la poitrine du patient, l'électrode avant (17) pouvant être positionnée adjacente au coussinet (5), à la plaque, au disque ou à l'élément similaire du moyen animé d'un mouvement de va-et-vient ou y étant intégrée de manière à ce qu'elle fasse face à la poitrine et à ce qu'elle puisse reposer contre celle-ci en combinaison avec le coussinet (5), la plaque, le disque ou l'élément similaire, et une électrode arrière (18) disposée de manière à pouvoir reposer contre le dos du patient.

4. Appareil selon la revendication 3, le moyen électrique (17, 18, 26) destiné à fournir une électrostimulation comprenant un moyen (26) destiné à analyser les signaux ECG captés par lesdites électrodes (17, 18) et acheminés jusqu'au moyen d'analyse de l'ECG.

5. Appareil selon les revendications 1 à 4, la fréquence de stimulation étant commandée pour suivre la fréquence de compression/décompression mécanique.
